(19) Europäisches Patentamt
European Patent Office
Office européen des brevets

(11) EP 3 346 972 B1

# (12) EUROPEAN PATENT SPECIFICATION

(45) Date of publication and mention of the grant of the patent:
**26.05.2021 Bulletin 2021/21**

(21) Application number: **16709948.0**

(22) Date of filing: **15.03.2016**

(51) Int Cl.:
***A61K 8/25*** (2006.01) ***A61K 8/362*** (2006.01)
***A61K 8/365*** (2006.01) ***A61K 8/41*** (2006.01)
***A61K 8/44*** (2006.01) ***A61Q 5/08*** (2006.01)
***A61Q 5/10*** (2006.01) ***A61K 8/19*** (2006.01)
***A61K 8/22*** (2006.01)

(86) International application number:
**PCT/EP2016/055587**

(87) International publication number:
**WO 2017/041907 (16.03.2017 Gazette 2017/11)**

## (54) PROCESS FOR TREATING HAIR

VERFAHREN ZUR HAARBEHANDLUNG

PROCÉDÉ DE TRAITEMENT DES CHEVEUX

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.09.2015 EP 15184312**

(43) Date of publication of application:
**18.07.2018 Bulletin 2018/29**

(73) Proprietor: **Kao Germany GmbH**
**64297 Darmstadt (DE)**

(72) Inventors:
- **NÖCKER, Bernd**
  **64297 Darmstadt (DE)**
- **BREAKSPEAR, Steven**
  **64297 Darmstadt (DE)**
- **BAUER, Peter**
  **64297 Darmstadt (DE)**
- **WOOD, Jonathan**
  **64297 Darmstadt (DE)**

(56) References cited:
**EP-A1- 2 191 812**
**EP-A1- 2 277 498**
**EP-A1- 2 338 470**
**EP-A1- 2 471 502**
**WO-A1-2011/003553**
**FR-A1- 2 958 160**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 346 972 B1

## Description

The present invention relates to a process for treating hair, especially human hair, for improved and milder oxidative treatment.

Oxidative hair treatments in the form of bleaching and/or dyeing have been used for many decades in order to permanently change the hair color of human hair. It involves the application of a strongly oxidative composition comprising the dyestuff precursors and optionally coupling substances onto hair and leaving it for a certain period of time usually at elevated temperatures in order to allow for penetration of the relatively small uncolored dye precursors into the hair (EP2338470). In combination with the action of strong oxidizing agents, the dye precursors polymerize to larger molecules so that they may not be easily eluted from the hair fiber with subsequent treatment and at the same time the effect of the oxidizing agent is to lighten the hair color to provide a relatively homogeneous dyeing base, or in the absence of the dyes in order to bleach the hair by oxidatively degrading the natural hair color melanin (EP2471502, WO2011/003553). Since the process involves the use of strong oxidative compositions, the hair fiber itself is negatively affected by such a treatment and it consequently loses certain natural cosmetic properties such as its strength against breaking, its natural elasticity, its natural shine and natural soft feel upon touching.

Moreover, the to be dyed and/or bleached hair is not always homogeneous in its physicochemical status as it may be damaged due to previous chemical treatments such as dyeing and permanently shaping and/or environmental effects. This often leads to inhomogeneous dyeing/bleaching performance and, therefore, consumers' dissatisfaction. There is, therefore, a great need for milder and more effective dyeing compositions which overcome one or more of the above mentioned problems.

Recently in a series of patent applications (US2015/0034119, US2015/0037270, WO2015/017768) methods are published which claim benefits of the combined use of a bismaleate based binding agent in hair chemical treatments such as oxidative hair dyeing, permanently shaping and bleaching for improving the hair structure. The publications are silent on the core of the present invention.

After a long research and careful considerations of the consumers' needs, the inventors of the present invention have unexpectedly found out that when commonly used oxidative treatment compositions are mixed with another composition comprising predominantly carboxylic acids, the dyeing and/or bleaching effects of the compositions are improved, homogeneous dyeing/bleaching of hair fibers is achieved and natural cosmetic properties of hair is maintained.

Therefore, the first object of the present invention is a process for treating hair, especially human hair, comprising the steps of

- application onto hair of a ready-to-use composition obtained by mixing the compositions A, B and C which are being kept separately until immediately before application onto hair at a weight ratio of A:B:C in the range of 1:2:0.2 to 1:1:1,
- leaving it on the hair for a period of 1 min to 45 min,
- rinsing it off from the hair, and
- optionally washing the hair with a cleansing composition and drying,

wherein the composition A is a composition comprising one or more alkalizing agents and having a pH ranging from 7.5 to 12,
wherein the composition B is an aqueous composition comprising one or more oxidizing agents and has a pH in the range of 1.5 to 5,
wherein the composition C comprises

i. one or more carboxylic acids having three or more carboxyl groups and/or their salts selected from citric acid, ethylenediamine tetraacetic acid (EDTA), pyromellitic acid and glutamate diacetate, and
ii. one or more additional organic acid and/or their salts having one or two carboxyl groups selected from acetic acid, malic acid, lactic acid, glycolic acid, tartaric acid, formic acid, oxalic acid, malonic acid, succinic acid, glutaric acid, adipic acid, maleic acid, and fumaric acid,

wherein the composition C comprises the acids of i) and ii) and/or their salts at a total concentration of 10% to 100% by weight calculated to the total of composition C, wherein the ready-to-use composition has an alkaline pH in the range from 8 to 11 and comprises the acids and/or their salts at a total concentration in the range from 1% to 10% by weight calculated to the total of the ready-to-use composition.

The second object is a kit for hair, especially human hair, comprising the compositions A, B and C as defined above.

The composition A comprises one or more alkalizing agents, preferably selected from sodium metasilicate, ammonia, alkyl- or alkanolamines according to the general structure

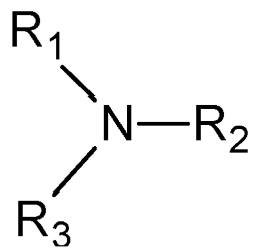

wherein $R_1$, $R_2$, and $R_3$ are same or different H, from $C_1$ to $C_4$, $C_3$ to $C_4$ unsaturated alkyl, $C_3$ to $C_4$ branched alkyl, $C_1$ to $C_4$ hydroxyl alkyl, $C_3$ to $C_4$ unsaturated hydroxyl alkyl, $C_3$ to $C_4$ branched hydroxyl alkyl, with the condition that at least one of $R_1$, $R_2$, or $R_3$ is different from H, wherein the alkalizing agents preferably selected from ammonia, monoethanolamine, and aminomethyl-propanol, and particularly suitable one is aminomethyl-propanol.

The alkalizing agent may be comprised in the composition A at a total concentration of 1% to 20%, preferably 1% to 17.5%, more preferably 2% to 15% and most preferably 2.5% to 13% by weight calculated to the total of the composition A.

The pH of the composition A is in the range of 7.5 to 12, preferably 9 to 11, more preferably 9 to 10.5 and most preferably 9.5 to 10.5.

The composition B is an aqueous composition and comprises one or more oxidizing agent(s). The oxidizing agents suitable are hydrogen peroxide, urea peroxide, melamin peroxide or perborate salts. The most preferred is hydrogen peroxide. The composition B may comprise one or more oxidizing agents at a total concentration of 1% to 20% by weight, preferably 2% to 15%, more preferably 2% to 12% and most preferably 3% to 12% by weight, calculated to the total of composition B. The composition B may be in the form of a solution, thickened gel or an emulsion.

Emulsion form is particularly preferred.

The pH of the composition B is in the range of 1.5 to 5.

The composition C comprises

i. one or more carboxylic acids having three or more carboxyl groups and/or their salts selected from citric acid, ethylenediamine tetraacetic acid (EDTA), pyromellitic acid and glutamate diacetate, and

ii. one or more additional organic acid and/or their salts having one or two carboxyl groups selected from acetic acid, malic acid, lactic acid, glycolic acid, tartaric acid, formic acid, oxalic acid, malonic acid, succinic acid, glutaric acid, adipic acid, maleic acid, and fumaric acid.

Suitable carboxylic acids with three or more carboxyl groups and/or their salts are citric acid, ethylenediamine tetraacetic acid (EDTA), pyromellitic acid and glutamate diacetate. The ethylenediamine tetraacetic acid (EDTA) and/or its salts such as monosodium, disodium, trisodium and tetrasodium salts are the most preferred ones. Suitable organic acids with one or two carboxyl groups and/or their salts are acetic acid, malic acid, lactic acid, glycolic acid, tartaric acid, formic acid, oxalic acid, malonic acid, succinic acid, glutaric acid, adipic acid, maleic acid, and fumaric acid. The composition C comprises as the second acid one or more organic acids having one or two carboxyl groups and the most preferred acid is malic acid and/or its salts such as sodium, potassium and ammonium salts.

The composition C comprises the two acids at a total concentration in the range of 10% to 100% by weight, preferably 12.5% to 90% by weight, more preferably 12.5% to 75% by weight and most preferably 12.5% to 60% by weight, calculated to the total of the composition C.

The two acids may be comprised in the composition C at a weight ratio of first acid (i) to second acid (ii) in the range from 10:1 to 1:250, preferably from 5:1 to 1:150, and more preferably from 2:1 to 1:100 and most preferably 1:50.

The composition C may be in the form of a powder, a dispersion, an emulsion or a solution. In a preferred embodiment of the present invention the composition C is an aqueous composition and preferably has a pH in the range of 1 to 5, preferably 2 to 4, more preferably in the range of 2.5 to 3.6. In the case that the pH must be adjusted to a certain value, the composition C comprises one or more alkalizing agents preferably selected from ammonia, alkyl- or alkanolamines according to the general structure disclosed above. Particularly preferred alkalizing agent is aminomethylpropanol.

The alkalizing agent may be comprised in the composition C at a total concentration of 1% to 20%, preferabl 1% to 17.5%, more preferably 2% to 15% and most preferably 2.5% to 13% by weight calculated to the total of the composition C.

In a further preferred embodiment of the present invention, the composition C comprises one or more thickening polymers selected from anionic, nonionic, cationic and amphoteric polymers, preferably selected from polymers with a viscosity of at least 500 mPa·s measured at a polymer concentration of 1% by weight in water and at 20°C with a Brookfield viscometer, such as at 10 rpm for 1 min, with an appropriate spindle.

Suitable polymers are cellulose polymers, alginates, polysaccharides and acrylic acid polymers, preferably methyl cellulose, ethyl cellulose, hydroxyethylcellulose, methylhydroxyethylcellulose, methylhydroxypropylcellulose, carboxymethyl cellulose, alginic acids, sodium alginates, ammonium alginates, calcium alginates, gum arabic, guar gum or xanthan gum, dehydroxanthan gum or acrylic acid polymers known with the CTFA adopted name Carbomer and its derivatives.

The preferred polymers are dehydroxanthan gum, xanthan gum, and polymeric anionic thickeners Carbomer and its derivatives. The particularly preferred thickening agent is dehydroxanthan gum. The thickening agents are preferably comprised in the composition C at a total concentration in the range of 0.1% to 5%, preferably, 0.2% to 3%, more preferably 0.25% to 2.5% and most preferably 0.3% to 2% by weight calculated to the total of the composition C.

In an embodiment of the present invention the composition C comprises one or more hair dyes. Suitably, the composition C comprises one or more oxidative dye precursors and optionally one or more coupling substances.

Suitable non-limiting examples of oxidative dye precursor classes are p-phenylendiamines, p-aminophenols, and heterocyclic compounds such as diaminopyrazols and substituted pyrimidines, and suitable coupling substances are resorcinols, m-aminophenols, m-phenylendiamines, pyridines and substituted derivatives, and naphthols.

Non-limiting examples of the oxidative dye precursor compounds are p-phenylenediamine, p-aminophenol, 2,5-diamino-toluene, 2-n-propyl or 2-ethyl-p-phenylenediamine, 2,6-di-methyl-p-phenylene- diamine, 2-(2,5-diaminophenyl) ethanol, 1-amino-4-bis-(2'-hydroxy-ethyl)amino- benzene, 2-(2-hydroxyethyl amino)-5-aminotoluene, 4,4'-diaminodiphenylamine, 4-aminodiphenylamine, 2-amino-5-N,N-diethyl aminotoluene, 4-amino-N-ethyl-N-isopropyl aniline, 2-chloro-p-phenylenediamine, 1-β-hydroxyethyl-2,5-diamino-4-chlorobenzene, 1-β-hydroxyethyl-2,5-diamino-4-methyl benzene, 2-methoxy-p-phenylenediamine, N,N-diethyl-p-phenylenediamine, 1-amino-4-β-methoxyethyl aminobenzene, 1-dimethyl-amino-4-aminobenzene, 1-hydroxy-2,5-diamino-4-methyl benzene, 1-hydroxymethyl-2,5-diaminobenzene, 1,3-dimethyl-2,5-diaminobenzene, 1,4-diamino isopropyl benzene and/or 1-amino-4-β-hydroxypropyl aminobenzene, 1-hydroxyethyl-4,5-diaminopyrazole, 3,4-diamino-5-hydroxypyrazole, 3,5-diaminopyrazole, 3,5-diamino pyrazol-1-carboxamide, 3-amino-5-hydroxypyrazole, 1-phenyl-2-methylpyrazole, 1-phenyl-3-methylpyrazole-5-one, 3,5-dimethylpyrazole, 3,5-dimethylpyrazole-1-methanol, 3,5-diamino-1,2,4-triazole, 4-aminophenol and the derivatives thereof such as 4-amino-3-methylphenol, 2-chloro-4-aminophenol, 2,6-dichloro-4-aminophenol, 2,4-diamino-phenol, 2,6-dibromo-4-aminophenol, tetraamino pyrimidines, triaminohydroxy pyrimidines, diaminomono- and -dihydroxy pyrimidines, aminotriazines, 5-amino salicylic acid and/or 1,2,4-triamino benzene, 2,5-diaminopyridine, 2,3-diaminopyridine, 2,6-diaminopyridine, 3-amino-2-methyl amino-6-methoxypyridine, 2-dimethyl-5-aminopyridine, 2-dimethyl aminoethyl-3-hydroxypyridine, 2-amino-4,6-dimethyl pyridine, 2-amino-3-hydroxypyridine, 3-amino-2(β-hydroxyethyl amino)-6-methoxy pyridine, 2,6-dimethyl amino-5-aminopyridine, 2-di(hydroxyethyl) amino-5-aminopyridine, 2-hydroxyethyl amino-5-aminopyridine, 4-hydroxy-2,5,6-triaminopyrimidine and/or the water-soluble salts thereof, and mixture thereof.

The total concentration of the dye precursors (developing substances) customarily ranges between 0.001% to 5%, preferably 0.01% to 4% and more preferably 0.05% to 3%, and most preferably 0.1% to 2% by weight, calculated to the total of the composition C.

The suitable non-limiting examples of the coupling substance if present in the composition A are 5-amino-2-methylphenol, 2-methyl-5-hydroxyethylaminophenol, 2,4,-diaminophenoxyethanol, 2-amino-4-hydroxyethylaminoanisol, 2-methyl-5-amino-6-chlorphenol, 1,3-bis(2,4-diaminophenoxy)propane, 2-bis(2-hydroxyethyl)aminotoluene, 2-amino-5-methylphenol, resorcinol, 2-methyl-resorcinol, 4-chlororesorcinol, 2-amino-4-chlorophenol, 5-amino-4-methoxy-2-methylphenol, 2-aminophenol, 3-amino-phenol, 1-methyl-2-hydroxy-4-aminobenzene, 3-N,N-dimethyl aminophenol, 2,6-dihydroxy-3,5-dimethoxypyridine, 5-amino-3-methylphenol, 6-amino-3-methylphenol, 1,3-diamino- benzene, 1-amino-3-(2'-hy-droxyethylamino)benzene, 1-amino-3-[bis(2'-hydroxy-ethyl) amino]benzene, α-naphthol, 4,6-dichlororesorcinol, 1,3-diamino-toluene, 4-hydroxy-1,2-methylenedioxy benzene, 1,5-dihydroxy naphthalene, 1,6-dihydroxy naphthalene, 1,7-dihydroxy naphthalene, 2,7-dihydroxy naphthalene, 1-hydroxy-2-methyl naphthalene, 4-hydroxy-1,2-methyldioxy benzene, 2,4-diamino-3-chlorophenol, 5-amino-2-methoxyphenol and/or 1-methoxy-2-amino-4-(2'-hydroxyethyl amino)benzene or the water-soluble salts thereof and mixture thereof.

In the composition A, the coupling substance(s) as reaction partners of the developing substance(s) may be present in approximately the same molecular proportions as the developing substances, i.e., at a total concentration in the range of 0.001% to 5%, preferably 0.01% to 4% and more preferably 0.05% to 3%, and most preferably 0.1% to 2% by weight, calculated to the total of the composition C.

In another preferred embodiment of the present invention the composition C comprise(s) one or more hair direct dyes. Suitable ones are cationic, anionic and nitro dyes. Plant dyes are also suitable for the compositions of the present invention. Suitable anionic direct dyes are Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C

Yellow No. 10, FD&C Red 2, FD&C Red 40, FD&C Red No. 4, FD&C Yellow No. 6, FD&C Blue 1, Food Black 1, Food Black 2, Disperse Black 9 and Disperse Violet 1 and their alkali metal salts such as sodium, potassium. Among those, the most preferred anionic dyestuffs are Acid Red 52, DC Violet 2, DC Red 33, DC Orange 4, DC Red 27, DC Yellow 10, HC Blue 18, HC Red 18, and HC Yellow 16. Suitable cationic dyes are in principle those available on the market for cosmetic hair colouring applications. For this purpose, special reference is made to the PCT application WO 95/15144 of Ciba-Geigy AG. Some examples to those are Basic Blue 6, Basic Blue 7, Basic Blue 9, Basic Blue 26, Basic Blue 41, Basic Blue 99, Basic Brown 4, Basic Brown 16, Basic Brown 17, Natural Brown 7, Basic Green 1, Basic Red 2, Basic Red 12 Basic Red 22, Basic Red 76, Basic Violet 1, Basic Violet 2, Basic Violet 3, Basic Violet 10, Basic Violet 14, Basic Yellow 57, Basic red 51, Basic Yellow 87, HC Blue 17 and Basic Orange 31. The most preferred ones are Basic red 51, Basic Yellow 87 and Basic Orange 31 sold by BASF, and HC Blue 17.

Suitable nitro dyes are HC Blue No.2, HC Blue No.4, HC Blue No.5, HC Blue No.6, HC Blue No.7, HC Blue No.8, HC Blue No.9, HC Blue No.10, HC Blue No.11, HC Blue No.12, HC Blue No.13, HC Brown No.1, HC Brown No.2, HC Green No.1, HC Orange No.1, HC Orange No.2, HC Orange No.3, HC Orange No.5, HC Red BN, HC Red No.1, HC Red No.3, HC Red No.7, HC Red No.8, HC Red No.9, HC Red No.10, HC Red No.11, HC Red No.13, HC Red No.54, HC Red No.14, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No.10, HC Yellow No.11, HC Yellow No.12, HC Yellow No.13, HC Yellow No.14, HC Yellow No.15, 2- Amino-6-chloro-4-nitrophenol, picramic acid, 1,2-Diamino-4-nitrobenzol, 1,4-Diamino-2-nitrobenzol, 3-Nitro-4-aminophenol, 1-Hydroxy-2-amino-3-nitrobenzol and 2-hydroxyethylpicramic acid.

Plant dyestuffs can also be used alone or in combination with synthetic direct-acting dyestuffs, for example henna (red or black), alkanna root, laccaic acid, indigo, logwood powder, madder root and rhubarb powder, etc.

The composition C may comprise one or more hair direct dye at a total concentration of 0.01% to 10%, preferably 0.05% to 7.5% and more preferably 0.1% to 5% by weight calculated to the total of the compositions C. The composition can also comprise a mixture of several direct dyes, i.e., an anionic, a cationic and/or nonionic ones. In such a case the dyes may be mixed at any ratio with each other.

In a further preferred embodiment of the present invention, the composition A is an anhydrous composition and comprises one or more persalts and sodium metasilicate as an alkalizing agent.

In a still further embodiment of the present invention, the composition C is an anhydrous composition and comprises one or more persalts and sodium metasilicate as an alkalizing agent.

The term anhydrous means that the composition does not comprise any added water. It should be noted that one or more ingredients of the anhydrous composition may comprise bound crystal water or residual moisture which is not covered by the term. Useful are sodium persulfate and potassium persulfate and ammonium persulfate, earth alkali peroxides such as magnesium peroxide, melamine peroxide or urea peroxide or phtholimidoperoxy hexanoic acid. The preferred persalts are sodium persulfate and potassium persulfate. The persalt may be comprised inthe composition A at a total concentration in the range of 10% to 80%, preferably 15% to 70%, more preferably 20% to 60% and most preferably 25% to 60% by weight, calculated to the total of composition A.

According to the invention, the anhydrous composition can also comprise one or more ammonium salts at a concentration from 0.1% to 10% by weight calculated to the total of composition A. Suitable ammonium salts are ammonium carbonate, ammonium hydrogen carbonate, ammonium carbamate, ammonium chloride, ammonium sulfate, ammonium phosphates, ammonium nitrate, ammonium bromide, ammonium iodide, ammonium thiosulfate, ammonium molybdate, ammonium vanadate, ammonium sulfamate, ammonium citrate, ammonium salicylate, ammonium valerate, ammonium tartarate, ammonium benzoate, ammonium acetate, ammonium formiate and ammonium lactate. Compositions may also comprise mixtures of ammonium salts.

Preferred thereof are the ammonium phosphates, such as ammonium dihydrogen phosphate, diammonium hydrogen phosphate, diammonium sodium phosphate, ammonium sodium hydrogen phosphate or ammonium disodium phosphate, ammonium chloride, ammonium sulfate and diammonium hydrogen citrate.

In the case the composition(s) A and/or C are anhydrous compositions, the alkalizing agent sodium metasilicate is comprised at a concentration from 1% to 20% by weight, calculated to the total of composition A and/or C.

Any of the compositions A, B or C may comprise one or more of the commonly used hair conditioning compounds. These compounds are for example fatty alcohols, surfactants such as anionic, nonionic, cationic and amphoteric ones, ubiquinones, ceramides, organic solvents, lipophilic ingredients such as vegetable oils, mineral oils, silicones, fatty acid fatty alcohol esters, preservatives, amino acids, and polyols. It should be noted that these compounds are optionally comprised in the any of the compositions and their incompatibility must be carefully considered prior to addition into the compositions.

Any of the compositions may comprise one or more fatty alcohols. In particular the compositions B and C may be aqueous composition and may further be in the form of an emulsion and then comprises preferably one or more fatty alcohols. Suitable fatty alcohols are the ones with the chain length of 14 to 22 C atoms which may be saturated or unsaturated, linear or branched which may as well be substituted. Non-limiting examples are myristyl alcohol, cetyl

alcohol, stearyl alcohol, behenyl alcohol and cetostearyl alcohol.

The total concentration of fatty alcohol may be in the range from 0.5% to 20%, preferably 1% to 15% by weight, calculated to the total of each composition.

Compositions of the process according to the present invention may comprise surfactants selected from anionic, nonionic, amphoteric and/or cationic surfactants. The anionic, nonionic, amphoteric surfactants are used generally as emulsifier or solubilizer whereas the cationic surfactants are at the same time particularly used as hair conditioners.

Anionic surfactants suitable are in principle known from the cleansing compositions. These are anionic surfactants of the sulfate, sulfonate, carboxylate and alkyl phosphate type, for example, the known $C_{10}$-$C_{18}$-alkyl sulfates, and in particular the respective ether sulfates, for example, $C_{12}$-$C_{14}$-alkyl ether sulfate, lauryl ether sulfate, especially with 1 to 4 ethylene oxide groups in the molecule, monoglyceride (ether) sulfates, fatty acid amide sulfates obtained by ethoxylation and subsequent sulfation of fatty acid alkanolamides, and the alkali salts thereof, as well as the salts of long-chain mono- and dialkyl phosphates.

Additional anionic surfactants useful within the scope of the invention are $\alpha$-olefin sulfonates or the salts thereof, and in particular alkali salts of sulfosuccinic acid semiesters, for example, the disodium salt of monooctyl sulfosuccinate and alkali salts of long-chain monoalkyl ethoxysulfosuccinates.

Suitable surfactants of the carboxylate type are alkyl polyether carboxylic acids and the salts thereof as well as alkyl amido polyether carboxylic acids and salts thereof. Such products have been known for some time and are on the market, for example, under the trade name "AKYPO®" and "AKYPO-SOFT®".

Also useful are $C_8$-$C_{20}$-acyl isethionates, alone or in admixture with other anionic surfactants, as well as sulfofatty acids and the esters thereof.

Further suitable anionic surfactants are also $C_8$-$C_{22}$-acyl aminocarboxylic acids or the water-soluble salts thereof. Especially preferred is N-lauroyl glutamate, in particular as sodium salt, as well as, for example, N-lauroyl sarcosinate, N-$C_{12}$-$C_{18}$-acyl asparaginic acid, N-myristoyl sarcosinate, N-oleoyl sarcosinate, N-lauroyl methylalanine, N-lauroyl lysine and N-lauroyl aminopropyl glycine, preferably in form of the water-soluble alkali or ammonium, in particular the sodium salts thereof, preferably in admixture with the above-named anionic surfactants.

It is also possible to use mixtures of several anionic surfactants.

Further surfactants suitable are nonionic surfactants. Non-limiting examples are long-chain fatty acid mono- and dialkanolamides, such as coco fatty acid mono- or diethanolamide and myristic fatty acid mono or diethanolamide, stearic acid mono or diethanolamide, alkyl polyglucosides with an alkyl group of 8 to 18 carbon atoms, and with 1 to 5 glucoside units, sorbitan esters, such as polyethylene glycol sorbitan stearic, palmitic, myristic and lauric acid esters, fatty acid polyglycol esters or polycondensates of ethyleneoxide and propyleneoxide, as they are on the market, for example, under the trade name "Pluronics$^R$", as well as fatty alcohol ethoxylates, $C_{10}$-$C_{22}$-fatty alcohol ethoxylates, known by the generic terms "Laureth", "Myristeth", "Oleth", "Ceteth", "Deceth", "Steareth" and "Ceteareth" according to the CTFA nomenclature, including addition of the number of ethylene oxide molecules, e.g., "Laureth-16": The average degree of ethoxylation thereby ranges between about 2.5 and about 100, preferably about 10 and about 30.

Suitable amphoteric surfactants are in particular the various known betaines such as alkyl betaines, fatty acid amidoalkyl betaines and sulfobetaines, for example, lauryl hydroxysulfobetaine; long-chain alkyl amino acids, such as cocoaminoacetate, cocoaminopropionate and sodium cocoamphopropionate and -acetate have also proven suitable.

Suitable cationic surfactants are according to the general structure

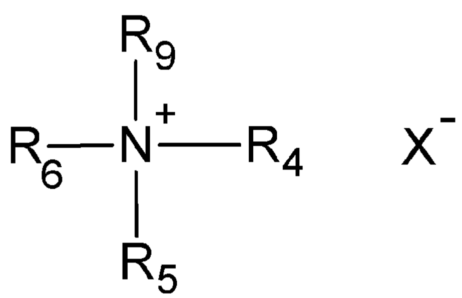

where $R_5$ is a saturated or unsaturated, branched or linear alkyl chain with 8-22 C atoms or

$$R_7\ CO\ NH\ (CH_2)_n$$

where $R_7$ is saturated or unsaturated, branched or linear alkyl chain with 7-21 C atoms and n has typical value of 1 - 4 or

$$R_8\ CO\ O\ (CH_2)_n$$

where $R_8$ is saturated or unsaturated, branched or linear alkyl chain with 7-21 C atoms and n has typical value of 1 - 4, and $R_4$ is H or unsaturated or saturated, branched or linear alkyl chain with 1 - 22 C atoms or

$$R_7\ CO\ NH\ (CH_2)_n$$

or

$$R_8\ CO\ O\ (CH_2)_n$$

where $R_7$, $R_8$ and n are same as above.
$R_9$ and $R_6$ are H or lower alkyl chain with 1 to 4 Carbon atoms, and X is typically chloride, bromide, methosulfate.

Typical examples of those ingredients are cetyl trimethly ammonium chloride, stearyl trimonium chloride, dipalmitoyl dimonium chloride, distearyl dimethyl ammonium chloride, stearamidopropyl trimonuim chloride, dioleoylethyl dimethyl ammonium methosulfate, dioleoylethyl hydroxyethylmonium methosulfate.

The concentration of one or more total surfactants in any of the compositions A, B, and/or C may be in the range of 0.1% to 20%, preferably 0.2% to 15% and most preferably 0.2% to 10% by weight, calculated to the total of each composition.

The compositions A, B, and/or C may further comprise lipophilic ingredients such as vegetable oils, for example, jojoba oil or any other; liquid paraffins, especially paraffinum perliquidum and paraffinum subliquidum; silicones for example linear polysiloxanes such as dimethicones with various consistency and dimethiconols, aminated silicones with primary, secondary, tertiary or quaternary ammonium groups such as amodimethicone, polysilicone 9, and quaternium 80, cyclic silicones such as cyclomethicones, arylated silicones such as phenyl trimethicone; fatty acid esters such as octyl palmitate, isocetyl palmitate, isopropyl palmitate and octyl stearate, $C_{10}$-to $C_{36}$-fatty acid triglycerides, as well as their mixtures. Total concentration of these lipophilic compounds may be in the range of 0.1% to 20% by weight, preferably from 1% to 15% by weight, and more preferably from 2% to 10% by weight, calculated to the total of each composition.

Composition A, B, and/or C can also comprise cationic polymers as conditioning and/or thickening agents. Those are cationic cellulose type polymers know as Polymer JR type from Amerchol such as Polyquaternium 10 or cationic guar gum known with trade name Jaguar from Rhône-Poulenc and chemically for example Guar hydroxypropyl trimonium chloride. Furthermore, chitosan and chitin can also be included in the compositions as cationic natural polymers.

Furthermore, it has been found suitable to use those cationic polymers known with their CTFA category name Polyquaternium. Typical examples of those Polyquaternium 4, Polyquaternium 6, Polyquaternium 7, Polyquaternium 10, Polyquaternium 11, Polyquaternium 16, Polyquaternium 22, Polyquaternium 24, Polyquaternium 28, Polyquaternium 30, Polyquaternium 37, Polyquaternium 36, Polyquaternium 46, Polyquaternium 67, and Polyquaternium 72.

Equally suitable polymers are known with their CTFA category name Quaternium are suitable. Those are for example Quaternium-8, Quaternium-14, Quaternium-15, Quaternium-18, Quaternium-22, Quaternium-24, Quaternium-26, Quaternium-27, Quaternium-30, Quaternium-33, Quaternium-53, Quaternium-60, Quaternium-61, Quaternium-72, Quaternium-78, Quaternium-80, Quaternium-81, Quaternium-82, Quaternium-83 and Quaternium-84.

The total concentration of cationic polymers may be in the range of 0.1% to 7.5% by weight, preferably 0.3% to 5% by weight and more preferably 0.5% to 2.5% by weight, calculated to the total of each composition

The compositions A, B, and/or C may comprise one or more ceramide compound, such as the one according to general formula

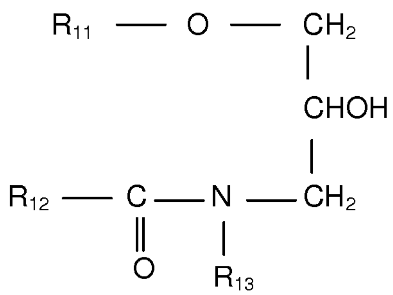

where $R_{11}$ and $R_{12}$ are independent from each other alkyl- or. alkenyl group with 10 to 22 carbon atoms, $R_{13}$ is alkyl or hydroxyl alkyl with 1 to 4 carbon atoms group and n is a number between 1 to 6, preferably 2 or 3. The preferred compound according to the above chemical structure is cetyl-PG-hydroxyethylpalmitamide. Concentration of ceramide type of compounds may range from 0.01% to 2%, preferably 0.01% to 1% by weight calculated to the total or each

composition.

The compositions A, B and/or C may comprise ubiquinone of the formula:

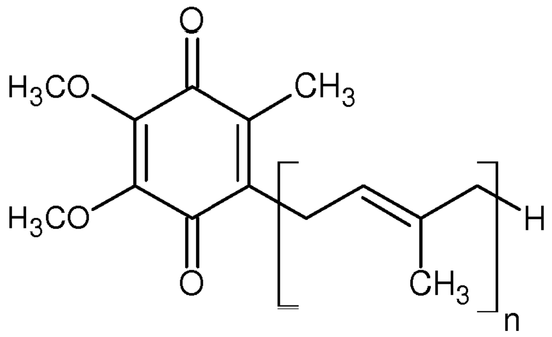

wherein n is a number from 1 to 10. The concentration of ubiquinone can vary between 0.001% and 10% by weight, calculated to the total of each composition.

The compositions A, B, and/or C may comprise one or more organic solvent such as 2-phenoxyethanol, benzyl alcohol, 2-phenylethanol and 2-benzyloxyethanol. Suitable aliphatic alcohols are ethanol, isopropanol, propanol, n-butanol, isobutanol, t-butanol and 1-pentanol. Concentration of one or more organic solvent may be in the range of 0.1% to 15%, preferably 0.5% to 12.5% and more preferably 1% to 10% and most preferably 1% to 7.5% by weight calculated to the total of each composition.

The compositions A, B, and/or C may further comprise one or more amino acids, preferably at a concentration in the range of 0.01% to 5%, preferably 0.1% to 3% and more preferably 0.2% to 2.5% and most preferably 0.25% to 2% by weight calculated to the total of each composition. Suitable ones are all of the known amino acids such as, arginine, alanine, asparagine, glutamine, glycine, histidine, leucine, lysine, methionine, phenylalanine, proline, serine, threonine, tryptophan, tyrosine and valine. The compositions A, B, and/or C may further comprise one or more polyol, preferably at a concentration in the range of 0.01% to 5%, preferably 0.1% to 3% and more preferably 0.2% to 2.5% and most preferably 0.25% to 2% by weight calculated to the total of each composition. Suitable ones are propylene glycol, dipropylene glycol, glycerine, panthenol and its derivatives.

The composition C may further comprise one or more reducing agents for improving long term stability of the oxidative dye precursors when they are present in the composition. The suitable ones are any of the known reducing agents such as sodium sulfite, ascorbic acid and its salts, thioglycolic acid and its salts. The concentration of the reducing agent in the composition C is typically in the range of 0.1% to 2% by weight calculated to the total of the composition C.

The compositions A, B, and/or C may further comprise any known preservatives if necessary.

The pH of the ready-to-use composition obtained by mixing the composition A, B, and C is in the range of 8 to 11, preferably 9 to 10.5, more preferably 9.5 to 10.5.

The following examples are to illustrate the invention, but not to limit it.

## Example 1

**The composition A**

| | % by weight |
|---|---|
| Monoethanol amine | 2.0 |
| Ammonium hydroxide | 8.0 |
| Fragrance, preservative | q.s. |
| Water | to 100 |

**The composition B**

| | % by weight |
|---|---|
| Hydrogen peroxide | 9.00 |
| Cetyl stearyl alcohol | 1.70 |
| Phosphoric acid | q.s. to pH 3.0 |

(continued)

| | % by weight |
|---|---|
| Sodium lauryl sulfate | 0.20 |
| Salicylic acid | 0.10 |
| Water | ad 100.00 |

**The composition C**

| | % by weight |
|---|---|
| EDTA tetrasodium salt | 1.0 |
| Malic acid | 13.0 |
| Aminomethylpropanol | 6.0 |
| Hydroxypropyl xanthan gum | 0.6 |
| Cetrimonium chloride | 0.1 |
| Preservative | q.s. |
| Water | to 100 |

The pH value of the above composition C was 3.5 ± 0.1.

The above three compositions A, B, and C were mixed at a weight ratio of A, B and C 1:2:0.2. The resulting composition had a pH of approximately 9.5. The composition was applied onto streaks of human hair and left on the hair for 30 min at ambient temperature, then rinsed off from the hair and the hair was shampooed and dried.

For comparative purposes, the same steps were carried out without using the composition C. Instead of composition C the same amount of water was added. The resulting composition had a pH of approximately 9.9. The composition was applied onto streaks of human hair and left on the hair for 30 min at ambient temperature, then rinsed off from the hair and the hair was shampooed and dried.

It was observed that the hair bleached with the inventive composition was much more cared in terms of its natural softness and elasticity. The shine of the hair dyed with the inventive composition was also better. Additionally, the streaks bleached with the inventive composition were more homogeneously bleached from root to tips, felt softer and easier to comb compared to the streaks treated with the comparative composition.

Similar results were obtained with the following compositions (composition C) when used with the compositions A and B of the Example 1.

**Example** 2

**The composition C**

| **Component** | **% by weight** |
|---|---|
| AMP | 6.0 |
| EDTA tetrasodium salt | 3.0 |
| Malic acid | 13.0 |
| Lactic acid | 4.0 |
| Hydroxypropyl xanthan gum | 0.6 |
| Polyquaternium-10 | 0.1 |
| Water | to 100 |

The pH value of the above composition C was 3.4 ± 0.1.

**Example 3:**

**The composition C**

| Component | % by weight |
|---|---|
| Monoethanolamine (MEA) | 2.7 |
| EDTA tetrasodium salt | 5.0 |
| Malic acid | 15.0 |
| Hydroxypropyl xanthan gum | 0.6 |
| Panthenol | 0.1 |
| Water | To 100 |

The pH value of the above composition C was 3.3 ± 0.1.

**Example 4:**

**The composition C**

| Component | % by weight |
|---|---|
| AMP | 6.0 |
| Citric acid | 5.0 |
| Maleic acid | 15.0 |
| Hydroxypropyl xanthan gum | 0.6 |
| Behenamidopropyl trimonium chloride | 0.2 |
| Water | to 100 |

The pH value of the above composition C was 1.4 ± 0.1.

**Example 5:**

**The composition C**

| Component | % by weight |
|---|---|
| MEA | 2.0 |
| Lactic acid | 15.0 |
| Citric acid | 6.0 |
| Hydroxypropyl xanthan gum | 0.6 |
| Polyquaternium-67 | 0.1 |
| Water | to 100 |

The pH value of the above composition C was 2.7 ± 0.1.

**Example 6**

**The composition C**

| | % by weight |
|---|---|
| EDTA tetrasodium salt | 1.0 |
| Malic acid | 13.0 |
| Aminomethylpropanol | 6.0 |
| Basic Red 51 | 1.00 |
| HC Red 18 | 1.00 |
| Hydroxypropyl xanthan gum | 0.6 |
| Cetrimonium chloride | 0.1 |
| Preservative | q.s. |
| Water | to 100 |

The pH value of the above composition C was 3.5 ± 0.1.

The hair was treated with the composition C from above using the compositions A and B of the Example 1 as described under the Example 1. It was observed that the hair was dyed effectively into intensive red color. The exclusion of the composition C resulted in loss of color brilliance and combability.

**Example 7**

**The composition C (powder)**

| | % by weight |
|---|---|
| EDTA tetrasodium salt | 7.0 |
| Malic acid | 93.0 |

1 g of the above composition C was added to the mixture of 10 g of composition A and 20 g of composition B (1 to 2) of the Example 1. After mixing thoroughly, the resulting composition was applied onto hair and rinsed off after leaving it on the hair for 30 min. It was observed that the hair as homogeneously bleached and felt natural upon touching.

**Example 8**

**The composition A (anhydrous composition)**

| | % by weight |
|---|---|
| Ammonium persulfate | 21.00 |
| Potassium persulfate | 36.60 |
| Sodium metasilicate | 11.00 |
| Diatomaceous Earth | 21.10 |
| Aerosil 380 | 1.00 |
| Liquid paraffin | 10.00 |

The above composition was prepared by combining all powder components and by mixing in a suitable mixer until homogeneity was reached.

The above composition A was mixed with the compositions B and C of the Example 1 at a weight ratio of A, B and C 1:2:0.2. The resulting composition had a pH of approximately 9.5. The composition was applied onto human hair and left on the hair for 30 min at ambient temperature, then rinsed off from hair and the hair was shampooed and dried.

For comparative purposes, the same steps were carried out without using the composition C. Instead of composition C the same amount of water was added. The resulting composition had a pH of approximately 9.9. The composition was applied onto human hair and left on the hair for 30 min at ambient temperature, then rinsed off from hair and the hair was shampooed and dried.

It was observed that the hair bleached with the inventive composition was much more cared as it has natural softness and elasticity. The shine of the hair bleached with the inventive composition was also better.

**Example 9**

| The composition C (anhydrous powder) | % by weight |
|---|---|
| EDTA tetrasodium salt | 5.0 |
| Malic acid | 15.0 |
| Ammonium persulfate | 21.00 |
| Potassium persulfate | 36.60 |
| Sodium metasilicate | 11.00 |
| Diatomaceous Earth | 1.10 |
| Aerosil 380 | 1.00 |
| Liquid paraffin | 10.00 |

The above composition C was mixed with the composition A and B of the Example 1 at a weight ratio of A, B, and C 1:2:1. The resulting composition had a pH of approximately 9.2. The composition was applied onto human hair and left on the hair for 30 min at ambient temperature, then rinsed off from hair and the hair was shampooed and dried. It was observed that the hair streak was bleached effectively and felt natural upon touching.

**Example 10**

| The composition C | % by weight |
|---|---|
| EDTA tetrasodium salt | 5.0 |
| Malic acid | 15.0 |
| Sodium lauryl sulphate | 1.5 |
| Propylene glycol | 2.0 |
| Cetyltrimonium chloride | 0.5 |
| 2,5,6-Triamino-4-hydroxypyrimidine sulfate | 0.01 |
| 2,5-Diaminotoluene sulfate | 0.55 |
| 4-Chlororesorcinol | 0.17 |
| Resorcinol | 0.05 |
| 3-Aminophenol | 0.03 |
| Sodium sulfite | 1.0 |
| Aminomethyl propanol | 2.0 |
| Ammonium hydroxide | q.s. to pH 10.0 |
| Fragrance, preservative | q.s. |
| Water | to 100 |

The above three compositions A, B and C were mixed at a weight ratio of A, B and C 1:2:1. The resulting composition had a pH of approximately 9.5. The composition was applied onto streaks of human hair and left on the hair for 30 min at ambient temperature, then rinsed off from the hair and the hair was shampooed and dried.

It was observed that the hair dyed with the composition was well cared in terms of its natural softness and elasticity. The hair was dyed homogeneously from root to tips, felt softer and possessed higher combability.

**Example 11**

**The composition C**

| | % by weight |
|---|---|
| EDTA monosodium salt | 1.0 |
| Malic acid | 13.0 |
| Aminomethylpropanol | 6.0 |
| Hydroxypropyl xanthan gum | 0.6 |
| Cetrimonium chloride | 0.1 |
| Preservative | q.s. |
| Water | to 100 |

The pH of the above composition C is approximately 3.1.

**Example 12**

**The composition C**

| | % by weight |
|---|---|
| EDTA disodium salt | 1.0 |
| Malic acid | 13.0 |
| Aminomethylpropanol | 6.0 |
| Hydroxypropyl xanthan gum | 0.6 |
| Cetrimonium chloride | 0.1 |
| Preservative | q.s. |
| Water | to 100 |

The pH of the above composition C is approximately 3.2.

**Example 13**

**The composition C**

| | % by weight |
|---|---|
| EDTA trisodium salt | 1.0 |
| Malic acid | 13.0 |
| Aminomethylpropanol | 6.0 |
| Hydroxypropyl xanthan gum | 0.6 |
| Cetrimonium chloride | 0.1 |
| Preservative | q.s. |
| Water | to 100 |

The pH of the above composition C is approximately 3.4.

**Example 14**

**The composition A**

| | % by weight |
|---|---|
| Cetearyl alcohol | 10.0 |
| Cocamide MEA | 4.0 |
| Sodium lauryl sulphate | 1.5 |

(continued)

| | % by weight |
|---|---|
| Propylene glycol | 2.0 |
| Cetyltrimonium chloride | 0.5 |
| 2,5,6-Triamino-4-hydroxypyrimidine sulfate | 0.01 |
| 2,5-Diaminotoluene sulfate | 0.55 |
| 4-Chlororesorcinol | 0.17 |
| Resorcinol | 0.05 |
| 3-Aminophenol | 0.03 |
| Sodium sulfite | 1.0 |
| Aminomethyl propanol | 2.0 |
| Ammonium hydroxide | q.s. to pH 10.0 |
| Fragrance, preservative | q.s. |
| Water | to 100 |

**The composition B**

| | % by weight |
|---|---|
| Hydrogen peroxide | 9.00 |
| Cetyl stearyl alcohol | 1.70 |
| Phosphoric acid | q.s. to pH 3.0 |
| Sodium lauryl sulfate | 0.20 |
| Salicylic acid | 0.10 |
| Water | ad 100.00 |

**The composition C**

| | % by weight |
|---|---|
| EDTA tetrasodium salt | 1.0 |
| Malic acid | 13.0 |
| Aminomethylpropanol | 6.0 |
| Hydroxypropyl xanthan gum | 0.6 |
| Cetrimonium chloride | 0.1 |
| Preservative | q.s. |
| Water | to 100 |

The pH value of the above composition D was 3.5 $\pm$ 0.1.

The compositions A, B and C were mixed at a weight ratio of A, B and C 2:1:0.2. The resulting composition had a pH of approximately 9.5. The composition was applied onto the bleached human hair streaks and left on the hair for 30 min at ambient temperature. Afterwards the hair was rinsed off, then shampooed with a Dualsenses Color shampoo, and dried (inventive process).

For comparative purposes, the same steps as described above were carried out without using the composition C. Instead of composition C the same amount of water was added. The resulting composition had a pH of approximately 9.9 (mixture of compositions A and B and water). The composition was applied onto bleached human hair as described above in subsequent order and left on the hair for 30 min at ambient temperature, rinsed off from hair, then shampooed with a Goldwell Dualsenses Color shampoo and dried (comparative process).

Color durability was investigated by incubating the hair streaks in a shaking bath for 20 min at 30°C. The water bath was filled with an aqueous solution of sodium laureth sulfate at a concentration of 3.5% by weight, calculated to the total of the solution. Upon incubation, the hair streaks were rinsed off with water and towel dried and dried with a hair drier. Color results were measured prior to incubation and after incubation by spectrophotometrical analysis with a Datacolor 45G CT instrument delivered from Datacolor Inc., Lawrenceville, NJ, USA. Based on the CIE*Lab color space results obtained by the measurements, $\Delta E_{ab}$ values for color difference were calculated according to equation (1):

$$\Delta E_{ab} = \sqrt{(L_2 - L_1) + (a_2 - a_1) + (b_2 - b_1)} \qquad \text{Equation 1}$$

The CIE*Lch values were measured with the same instrument and were reported for comparison purposes as well.

| Treatment group | Inventive Process | | | | | Comparative Process | | | | |
|---|---|---|---|---|---|---|---|---|---|---|
| | L | a | b | c | h | L | a | b | c | h |
| Before (1) | 41.42 | 35.57 | 27.78 | 45.13 | 38.00 | 42.15 | 36.56 | 29.47 | 46.96 | 38.87 |
| After (2) | 50.11 | 27.74 | 26.28 | 38.21 | 43.45 | 54.09 | 23.99 | 23.98 | 33.92 | 45.00 |
| | | | | | | | | | | |
| | Δ L | Δ a | Δ b | Δ c | Δ h | Δ L | Δ a | Δ b | Δ c | Δ h |
| | 8.70 | -7.82 | -1.50 | -6.92 | 5.45 | 11.94 | 12.57 | -5.48 | -13.03 | 4.26 |
| | | | | | | | | | | |
| **Δ Eab** | **11.8** | | | **-** | | **18.2** | | | - | |

The results clearly showed that the inventive process delivered lower $\Delta E_{ab}$ results compared to the comparative process which corresponds to a lower change and higher durability of the color upon incubation. Consequently the inventive process led to a more long-lasting color result. Furthermore, color vibrancy (c value) strongly dropped upon treatment of the streak with the comparative process by approximately 13 color units, whereas the inventive process stabilized the color vibrancy at about half of that value. In conclusion the presented data clearly showed the superior performance of the inventive process compared to the state-of-the-art process.

## Claims

**1.** A process for treating hair, especially human hair, comprising the steps of

- application onto hair of a ready-to-use composition obtained by mixing the compositions A, B and C which are being kept separately until immediately before application onto hair at a weight ratio of A:B:C in the range of 1:2:0.2 to 1:1:1,
- leaving it on the hair for a period of 1 min to 45 min,
- rinsing it off from the hair, and
- optionally washing the hair with a cleansing composition and drying,

wherein the composition A is a composition comprising one or more alkalizing agents and having a pH ranging from 7.5 to 12,
wherein the composition B is an aqueous composition comprising one or more oxidizing agents and has a pH in the range of 1.5 to 5,
wherein the composition C comprises

i. one or more carboxylic acids having three or more carboxyl groups and/or their salts selected from citric acid, ethylenediamine tetraacetic acid (EDTA), pyromellitic acid and glutamate diacetate, and
ii. one or more additional organic acid and/or their salts having one or two carboxyl groups selected from the group of acetic acid, malic acid, lactic acid, glycolic acid, tartaric acid, formic acid, oxalic acid, malonic acid, succinic acid, glutaric acid, adipic acid, maleic acid, and fumaric acid,

wherein the composition C comprises the acids of i) and ii) and/or their salts at a total concentration of 10% to 100% by weight calculated to the total of composition C,
wherein the ready-to-use composition has an alkaline pH in the range from 8 to 11 and comprises the acids and/or their salts at a total concentration in the range from 1% to 10% by weight calculated to the total of the ready-to-use composition.

**2.** The process according to claim 1 wherein the composition C comprises the first acid (i) and the second acid (ii) at

a weight ratio (i)/(ii) in the range of 10:1 to 1:250, preferably 5:1 to 1:150, and more preferably 2:1 to 1:100 and most preferably 1:50.

**3.** The process according to claims 1 to 2 wherein the composition C is a powder, dispersion, an emulsion or a solution.

**4.** The process according to any of the preceding claims wherein the carboxylic acid with 3 or more carboxyl groups is EDTA and/or its salts.

**5.** The process according to any of the preceding claims wherein the organic acid with one or two carboxyl groups is malic acid and/or its salts.

**6.** The process according to any of the preceding claims wherein the composition C comprises one or more hair dyes, selected from oxidative dye precursors, preferably selected from p-phenylendiamines, p-aminophenols, and heterocyclic diamines such as diaminepyrazols, and optionally one or more coupling substances, preferably selected from resorcinols, m-aminophenols, m-phenylendiamines, pyridines and substituted derivatives, and naphthols.

**7.** The process according to any of the preceding claims wherein the composition C comprises one or more hair direct dye selected from cationic, anionic and nitro dyes or their mixtures.

**8.** The process according any of the preceding claims wherein the composition C is an anhydrous composition and comprises one or more persalts and sodium metasilicate as an alkalizing agent.

**9.** The process according to claims 1 to 7 wherein the composition A is an anhydrous composition and comprises one or more persalts and sodium metasilicate as an alkalizing agent.

**10.** The process according to claims 1 to 7 wherein the composition C is an aqueous composition and preferably has a pH in the range from 1 to 5.

**11.** The process according to claims 6, 7, and 10 wherein the composition A and/or C comprise at least one alkalizing agent selected from ammonia, alkyl- or alkanolamines according to the general structure

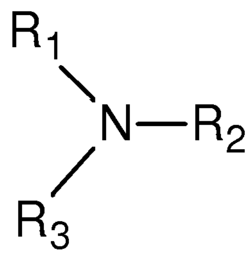

wherein $R_1$, $R_2$, and $R_3$ are same or different H, from $C_1$ to $C_4$, $C_3$ to $C_4$ unsaturated alkyl, $C_3$ to $C_4$ branched alkyl, $C_1$ to $C_4$ hydroxyl alkyl, $C_3$ to $C_4$ unsaturated hydroxyl alkyl, $C_3$ to $C_4$ branched hydroxyl alkyl, with the condition that at least one of $R_1$, $R_2$, or $R_3$ is different from H, wherein the alkalizing agent is preferably selected from ammonia, monoethanolamine, and aminomethylpropanol.

**12.** The process according to any of the preceding claims wherein the composition A and/or B and/or C comprises one or more ingredients selected from fatty alcohols, surfactants selected from anionic, nonionic, cationic and amphoteric ones, ubiquinones, ceramides, reducing agents, organic solvents, silicones such as linear polysiloxanes, aminated silicones, cyclic silicones, arylated silicones, antioxidants, preservatives, amino acids, polyols.

**13.** The process according to any of the preceding claims wherein the composition C comprises one or more thickening polymers selected from anionic, nonionic, cationic and amphoteric polymers, selected from polymers having a viscosity of at least 500 mPa·s measured at a polymer concentration of 1% by weight in water and at 20°C with a Brookfield viscometer, such as at 10 rpm for 1 minute, with an appropriate spindle, wherein the thickening polymer is preferably selected from hydroxypropyl xanthan gum, dehydroxanthan gum, xanthan gum, and polymeric anionic thickeners.

**14.** The process according to any of the preceeding claims wherein the carboxylic acid with three or more carboxyl

groups of composition C is EDTA and/or its salts and the additional organic acid having one or two carboxyl groups is malic acid and/or its salt.

**15.** Kit for hair comprising the compositions A, B, and C as defined in any of the preceding claims.

## Patentansprüche

**1.** Verfahren zum Behandeln von Haaren, insbesondere menschlichen Haaren, aufweisend die Schritte

- Aufbringen einer gebrauchsfertigen Zusammensetzung auf die Haare, die durch Vermischen der Zusammensetzungen A, B und C, welche bis unmittelbar vor dem Aufbringen auf die Haare getrennt gehalten werden, in einem Gewichtsverhältnis von A:B:C im Bereich von 1:2:0,2 bis 1:1:1 erhalten wird,
- Belassen selbiger auf den Haaren für eine Dauer von 1 min bis 45 min,
- Ausspülen selbiger aus den Haaren, und
- gegebenenfalls Waschen der Haare mit einer Reinigungszusammensetzung und Trocknen,

wobei die Zusammensetzung A eine Zusammensetzung ist, welche ein oder mehrere Alkalisierungsmittel aufweist und einen pH-Wert im Bereich von 7,5 bis 12 aufweist,
wobei die Zusammensetzung B eine wässrige Zusammensetzung ist, welche ein oder mehrere Oxidationsmittel aufweist und einen pH-Wert im Bereich von 1,5 bis 5 aufweist,
wobei die Zusammensetzung C aufweist:

i. eine oder mehrere Carbonsäuren mit drei oder mehr Carboxylgruppen und/oder deren Salze, ausgewählt aus Citronensäure, Ethylendiamintetraessigsäure (EDTA), Pyromellitsäure und Glutamatdiacetat, und
ii. eine oder mehrere zusätzliche organische Säuren und/oder deren Salze, welche eine oder zwei Carboxylgruppen aufweisen, ausgewählt aus der Gruppe von Essigsäure, Äpfelsäure, Milchsäure, Glykolsäure, Weinsäure, Ameisensäure, Oxalsäure, Malonsäure, Bernsteinsäure, Glutarsäure, Adipinsäure, Maleinsäure und Fumarsäure,

wobei die Zusammensetzung C die Säuren von i) und ii) und/oder deren Salze in einer Gesamtkonzentration von 10 Gewichts-% bis 100 Gewichts-% aufweist, bezogen auf die gesamte Zusammensetzung C,
wobei die gebrauchsfertige Zusammensetzung einen alkalischen pH-Wert im Bereich von 8 bis 11 aufweist und die Säuren und/oder deren Salze in einer Gesamtkonzentration im Bereich von 1 Gewichts-% bis 10 Gewichts-% aufweist, bezogen auf die gesamte gebrauchsfertige Zusammensetzung.

**2.** Verfahren nach Anspruch 1, wobei die Zusammensetzung C die erste Säure (i) und die zweite Säure (ii) in einem Gewichtsverhältnis (i)/(ii) im Bereich von 10:1 bis 1:250, vorzugsweise 5:1 bis 1:150 und mehr bevorzugt 2:1 bis 1:100 und am meisten bevorzugt 1:50 aufweist.

**3.** Verfahren nach Anspruch 1 bis 2, wobei die Zusammensetzung C ein Pulver, eine Dispersion, eine Emulsion oder eine Lösung ist.

**4.** Verfahren nach einem der vorhergehenden Ansprüche, wobei es sich bei der Carbonsäure mit 3 oder mehr Carboxylgruppen um EDTA und/oder deren Salze handelt.

**5.** Verfahren nach einem der vorhergehenden Ansprüche, wobei es sich bei der organischen Säure mit einer oder zwei Carboxylgruppen um Äpfelsäure und/oder deren Salze handelt.

**6.** Verfahren nach einem der vorhergehenden Ansprüche, wobei die Zusammensetzung C ein oder mehrere Haarfärbemittel, ausgewählt aus Vorstufen von Oxidativfarbstoffen, vorzugsweise ausgewählt aus p-Phenylendiaminen, p-Aminophenolen und heterocyclischen Diaminen, z.B. Diaminopyrazolen, und gegebenenfalls eine oder mehrere Kupplungssubstanzen aufweist, vorzugsweise ausgewählt aus Resorcinolen, m-Aminophenolen, m-Phenylendiaminen, Pyridinen und substituierten Derivativen und Naphtholen.

**7.** Verfahren nach einem der vorhergehenden Ansprüche, wobei die Zusammensetzung C ein oder mehrere Haardirektfärbemittel aufweist, ausgewählt aus kationischen Farbstoffen, anionischen Farbstoffen und Nitrofarbstoffen oder deren Gemischen.

**8.** Verfahren nach einem der vorhergehenden Ansprüche, wobei die Zusammensetzung C eine wasserfreie Zusammensetzung ist und ein oder mehrere Persalze und Natriummetasilicat als ein Alkalisierungsmittel aufweist.

**9.** Verfahren nach Anspruch 1 bis 7, wobei die Zusammensetzung A eine wasserfreie Zusammensetzung ist und ein oder mehrere Persalze und Natriummetasilicat als ein Alkalisierungsmittel aufweist.

**10.** Verfahren nach Anspruch 1 bis 7, wobei die Zusammensetzung C eine wässrige Zusammensetzung ist und vorzugsweise einen pH-Wert im Bereich von 1 bis 5 aufweist.

**11.** Verfahren nach Anspruch 6, 7 und 10, wobei die Zusammensetzung A und/oder C mindestens ein Alkalisierungsmittel aufweist, ausgewählt aus Ammoniak, Alkyl- oder Alkanolaminen gemäß der allgemeinen Struktur

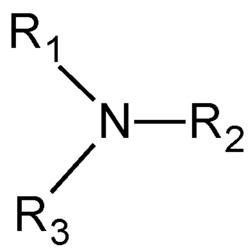

,

wobei $R_1$, $R_2$ und $R_3$ gleich oder verschieden sind und für H, $C_1$- bis $C_4$-, ungesättigtes $C_3$- bis $C_4$-Alkyl, verzweigtes $C_3$- bis $C_4$-Alkyl, $C_1$- bis $C_4$-Hydroxyalkyl, ungesättigtes $C_3$- bis $C_4$-Hydroxyalkyl, verzweigtes $C_3$- bis $C_4$-Hydroxyalkyl stehen, mit der Maßgabe, dass mindestens eines aus $R_1$, $R_2$ oder $R_3$ für anderes als H steht, wobei das Alkalisierungsmittel vorzugsweise aus Ammoniak, Monoethanolamin und Aminomethylpropanol ausgewählt ist.

**12.** Verfahren nach einem der vorhergehenden Ansprüche, wobei die Zusammensetzung A und/oder B und/oder C einen oder mehrere Inhaltsstoffe aufweist, ausgewählt aus Fettalkoholen, Tensiden, ausgewählt aus anionischen, nichtionischen, kationischen und amphoteren, Ubichinonen, Ceramiden, Reduktionsmittteln, organischen Lösungsmitteln, Silikonen, z.B. linearen Polysiloxanen, aminierten Silikonen, cyclischen Silikonen, arylierten Silikonen, Antioxidationsmitteln, Konservierungsmitteln, Aminosäuren, Polyolen.

**13.** Verfahren nach einem der vorhergehenden Ansprüche, wobei die Zusammensetzung C einen oder mehrere verdickende Polymere aufweist, ausgewählt aus anionischen, nichtionischen, kationischen und amphoteren Polymeren, ausgewählt aus Polymeren mit einer Viskosität von mindestens 500 mPa s, gemessen bei einer Polymerkonzentration von 1 Gewichts-% in Wasser und bei 20 °C mit einem Brookfield-Viskosimeter, z.B. mit 10 U/min für 1 Minute, mit einer geeigneten Spindel, wobei das verdickende Polymer vorzugsweise aus Hydroxypropylxanthan, Dehydroxanthan, Xanthan und polymeren anionischen Verdickungsmitteln ausgewählt ist.

**14.** Verfahren nach einem der vorhergehenden Ansprüche, wobei es sich bei der Carbonsäure mit drei oder mehr Carboxylgruppen der Zusammensetzung C um EDTA und/oder deren Salze handelt und bei der zusätzlichen organischen Säure mit einer oder zwei Carboxylgruppen um Äpfelsäure und/oder deren Salze handelt.

**15.** Kit für Haare, welches die Zusammensetzungen A, B und C aufweist, wie in einem der vorhergehenden Ansprüche definiert.

**Revendications**

**1.** Procédé de traitement des cheveux, notamment des cheveux humains, comprenant les étapes

- d’application d’une composition prête à l’emploi sur les cheveux, obtenue en mélangeant les compositions A, B et C qui sont conservées séparément jusqu’à immédiatement avant l’application sur les cheveux dans un rapport en poids A : B : C dans la plage de 1 : 2 : 0,2 à 1 : 1 : 1,
- de repos de celle-ci sur les cheveux pendant une durée de 1 min à 45 min,
- de retrait de celle-ci des cheveux par rinçage, et
- éventuellement, de lavage des cheveux avec une composition nettoyante, et de séchage,

où la composition A est une composition comprenant un ou plusieurs agents d'alcalinisation et ayant un pH dans la plage de 7,5 à 12,
où la composition B est une composition aqueuse comprenant un ou plusieurs agents d'oxydation et a un pH dans la plage de 1,5 à 5,
où la composition C comprend

i. un ou plusieurs acides carboxyliques ayant trois ou plus de trois groupements carboxyles, et/ou leurs sels, choisis parmi l'acide citrique, l'acide éthylène diamine tétra acétique (EDTA), l'acide pyroméllitique et le diacétate de glutamate, et
ii. un ou plusieurs acides organiques supplémentaires, et/ou leurs sels, ayant un ou deux groupements carboxyles, choisis dans le groupe de l'acide acétique, de l'acide malique, de l'acide lactique, de l'acide glycolique, de l'acide tartrique, de l'acide formique, de l'acide oxalique, de l'acide malonique, de l'acide succinique, de l'acide glutarique, de l'acide adipique, de l'acide maléique, et de l'acide fumarique,

où la composition C comprend les acides selon i) et ii) et/ou leurs sels à une concentration totale de 10 % à 100 % en poids calculée par rapport au total de la composition C,
où la composition prête à l'emploi a un pH alcalin dans la plage de 8 à 11 et comprend les acides et/ou leurs sels à une concentration totale dans la plage de 1 % à 10 % en poids calculée par rapport au total de la composition prête à l'emploi.

2. Procédé selon la revendication 1, dans lequel la composition C comprend le premier acide (i) et le second acide (ii) dans un rapport en poids (i)/(ii) dans la plage de 10 : 1 à 1 : 250, de préférence de 5 : 1 à 1 : 150, et plus préférentiellement de 2 : 1 à 1 : 100, et idéalement de 1 : 50.

3. Procédé selon les revendications 1 à 2, dans lequel la composition C est une poudre, une dispersion, une émulsion ou une solution.

4. Procédé selon l'une quelconque des revendications précédentes, dans lequel l'acide carboxylique avec 3 ou plus de trois groupements carboxyles est l'EDTA et/ou ses sels.

5. Procédé selon l'une quelconque des revendications précédentes, dans lequel l'acide organique avec un ou deux groupements carboxyles est l'acide malique et/ou ses sels.

6. Procédé selon l'une quelconque des revendications précédentes, dans lequel la composition C comprend un ou plusieurs colorants pour cheveux, choisis parmi des précurseurs de colorants oxydatifs, de préférence choisis parmi les p-phénylène diamines, les p-amino phénols, et des diamines hétérocycliques telles que des diamine pyrazoles, et éventuellement une ou plusieurs substances de couplage, de préférence choisies parmi les résorcinols, les m-amino phénols, les m-phénylène diamines, les pyridines et les dérivés substitués, et les naphtols.

7. Procédé selon l'une quelconque des revendications précédentes, dans lequel la composition C comprend un ou plusieurs colorants directs pour cheveux choisis parmi des colorants cationiques, anioniques et nitro, ou leurs mélanges.

8. Procédé selon l'une quelconque des revendications précédentes, dans lequel la composition C est une composition anhydre et comprend un ou plusieurs persels et du métasilicate de sodium en tant qu'agent d'alcalinisation.

9. Procédé selon les revendications 1 à 7, dans lequel la composition A est une composition anhydre et comprend un ou plusieurs persels et du métasilicate de sodium en tant qu'agent d'alcalinisation.

10. Procédé selon les revendications 1 à 7, dans lequel la composition C est une composition aqueuse et de préférence a un pH dans la plage de 1 à 5.

11. Procédé selon les revendications 6, 7, et 10, dans lequel les compositions A et/ou C comprennent au moins un agent d'alcalinisation choisi parmi l'ammoniac, les alkylamines ou alcanolamines selon la structure générale

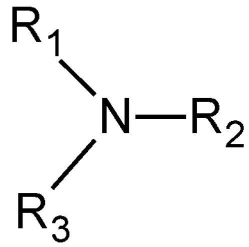

où $R_1$, $R_2$, et $R_3$ sont de manière identique ou différente l'atome H, un groupement alkyle insaturé de $C_1$ à $C_4$, de $C_3$ à $C_4$, un groupement alkyle ramifié en $C_3$ à $C_4$, un groupement alkyle hydroxylé en $C_1$ à $C_4$, un groupement alkyle hydroxylé insaturé en $C_3$ à $C_4$, un groupement alkyle hydroxylé ramifié en $C_3$ à $C_4$, à condition qu'au moins un parmi $R_1$, $R_2$, ou $R_3$ soit différent de l'atome H, où l'agent d'alcalinisation est de préférence choisi parmi l'ammoniac, la mono éthanolamine, et l'amino méthyl propanol.

**12.** Procédé selon l'une quelconque des revendications précédentes, dans lequel les compositions A, et/ou B, et/ou C comprennent un ou plusieurs constituants choisis parmi des alcools gras, des tensioactifs choisis parmi les tensioactifs anioniques, non ioniques, cationiques et amphotères, des ubiquinones, des céramides, des agents de réduction, des solvants organiques, des silicones tels que les polysiloxanes linéaires, des silicones aminés, des silicones cycliques, des silicones arylés, des antioxydants, des conservateurs, des acides aminés, des polyols.

**13.** Procédé selon l'une quelconque des revendications précédentes, dans lequel la composition C comprend un ou plusieurs polymères épaississants choisis parmi des polymères anioniques, non ioniques, cationiques et amphotères, choisis parmi des polymères ayant une viscosité d'au moins 500 mPas mesurée à une concentration de polymère de 1 % en poids dans l'eau, et à 20 °C avec un viscosimètre de Brookfield, comme à 10 tpm pendant 1 minute, avec une broche appropriée, où le polymère d'épaississement est de préférence choisi parmi la gomme hydroxypropyl xanthane, la gomme déhydroxanthane, la gomme xanthane, et des épaississants polymériques anioniques.

**14.** Procédé selon l'une quelconque des revendications précédentes, dans lequel l'acide carboxylique avec trois ou plus de trois groupements carboxyles de la composition C est l'EDTA et/ou ses sels et l'acide organique supplémentaire ayant un ou deux groupements carboxyles est l'acide malique et/ou son sel.

**15.** Kit pour cheveux comprenant les compositions A, B, et C selon l'une quelconque des revendications précédentes.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 2338470 A
- EP 2471502 A
- WO 2011003553 A
- US 20150034119 A
- US 20150037270 A
- WO 2015017768 A
- WO 9515144 A